# EUROPÄISCHE PATENTSCHRIFT

(11) **EP 1 849 759 B1**
(45) Veröffentlichungstag und Bekanntmachung des Hinweises auf die Patenterteilung: **18.11.2009**
(21) Anmeldenummer: 06111219.9
(22) Anmeldetag: 15.03.2006
(51) Int. Cl.: C07C 33/14, C07C 49/553, C07C 47/44, C07D 303/04, A61K 8/34, A61Q 13/00

(54) **Sandelriechstoffe**
Sandal odorants
Parfums de santal

(30) Priorität: 10.06.2005 DE 102005026801
(43) Veröffentlichungstag der Anmeldung: 31.10.2007
(73) Patentinhaber: Symrise GmbH & Co. KG, 37603 Holzminden (DE)
(72) Erfinder: Eh, Marcus, 37603, Holzminden (DE)
(74) Vertreter: Eisenführ, Speiser & Partner

(56) Entgegenhaltungen:
- EP-A2- 0 373 556
- US-A- 5 189 013

## Beschreibung

Die vorliegende Erfindung betrifft neue 4-(2,2,3-Trimethyl-cyclopent-3-enyl)-cyclohex-3-enylalkanole (vergleiche die Formel (la) weiter unten) und 3-(2,2,3-Trimethyl-cyclopent-3-enyl)-cyclohex-3-enylalkanole (vergleiche die Formel (Ib) weiter unten). Die Erfindung betrifft zudem Mischungen, welche die erfindungsgemäßen Verbindungen umfassen, die Verwendung einer erfindungsgemäßen Verbindung oder Mischung als Riechstoff sowie entsprechende parfümierte Produkte. Schließlich betrifft die Erfindung auch Verfahren zur Herstellung der erfindungsgemäßen Verbindungen und Mischungen sowie bestimmte Zwischenprodukte, die in den erfindungsgemäßen Herstellungsverfahren Verwendung finden.

In der Riechstoffindustrie besteht ein nachhaltiges Interesse an der Entwicklung von neuen Riechstoffen, um die Kreation von neuen Parfümölen für die alkoholische und für die funktionelle Parfümerie zu ermöglichen. Verbindungen mit holzigem Geruch sind hierbei unverzichtbare Komponenten in der Duftstoffindustrie. Eine besonders wertvolle Klasse dieser holzigen Riechstoffe sind Verbindungen mit Sandelholzgeruch. Strukturell zeichnen sich Verbindungen mit Sandelholzgeruch häufig durch ein 4-(2,2,3-Trimethyl-cyclopent-3-enyl)-butan-1-ol-Grundgerüst aus, wobei die Butan-1-ol-Seitenkette gesättigt oder einfach ungesättigt sowie ein- oder mehrfach methylsubstituiert sein kann. Einige Vertreter dieser Klasse von Riechstoffen sind 2-Methyl-4-(2,2,3-trimethyl-cyclopent-3-enyl)-butan-1-ol (II) (Brahmanol^{®}, Symrise GmbH & Co. KG), 2-Ethyl-4-(2,2,3-trimethyl-cyclopent-3-enyl)-but-2-en-1-ol (III) (Sandranol^{®}, Symrise GmbH & Co. KG), 3-Methyl-5-(2,2,3-trimethyl-cyclopent-3-enyl)-pent-4-en-2-ol (IV) (Ebanol^{®}, Givaudan S.A.) und 3,3-Dimethyl-5-(2,2,3-trimethyl-cyclopent-3-enyl)-pent-4-en-2-ol (V) (Polysantol^{®}, Firmenich S.A.).

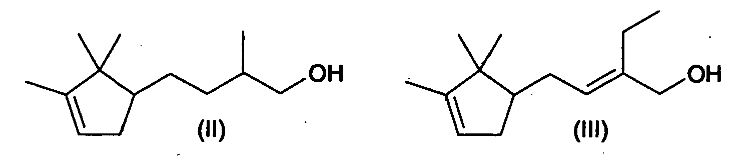

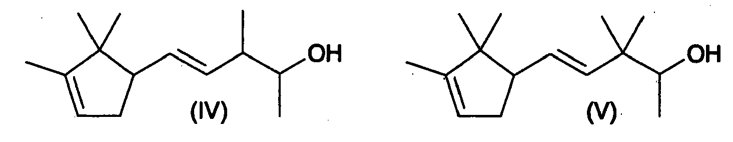

Die hier gezeigten Verbindungen (II) bis (V) zeichnen sich durch einen starken Sandelholzgeruch aus, der (a) in der Stärke und (b) in weiteren Geruchsaspekten der einzelnen Verbindungen (II) - (V) untereinander variiert.

Die US 5189013 offenbart Verbindungen des Typs (VI), das heißt Verbindungen, in denen anstelle einer Alkanol- bzw. Alkenol-Seitenkette (wie in den Verbindungen (II) bis (V)) ein gesättigter oder ungesättigter Cyclohexanon- oder Cyclohexanol-Ring vorhanden ist.

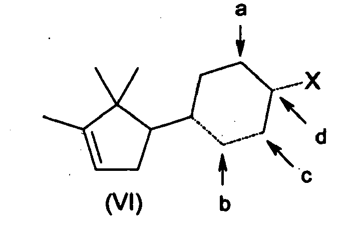

In der Formel (VI) bedeutet die exocyclische gestrichelte Linie eine Einfachbindung (X ist dann OH) oder eine Doppelbindung (X ist dann O). Der sechsgliedrige Ring kann in den Positionen a, b, c und d eine oder mehrere Methylsubstituenten besitzen. Die in der US 5189013 offenbarten Verbindungen weisen zumeist einen allenfalls schwachen Sandelholzgeruch auf. Lediglich hinsichtlich der Verbindung 1,2-Dimethyl-4-(2',2',3'-trimethyl-3'-cyclopenten-1'-yl)-2-cyclohexen-1-ol wird ausgeführt, dass diese einen Geruch besitze, dessen Charakter dem des natürlichen Sandelholzes ähnlich ist. Die genannte Verbindung ist eine Verbindung der Formel (VI), in der X für OH steht und in welcher der sechsgliedrige Ring eine Doppelbindung in Position 2 besitzt, das heißt zwischen den Pfeilen c und b.

In Chemistry & Biodiversity, 1, 980-1021, 2004 werden Verbindungen des Typs (VII) und (VIII) beschrieben.

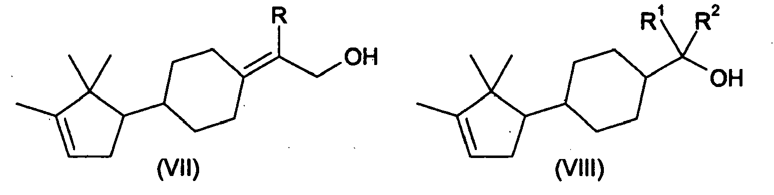

Hierbei können die Reste R, R¹ und R² H oder -CH₃ sein. Die offenbarten Verbindungen zeigen jedoch in keinem Fall einen typischen Sandelholzgeruch; falls ein Sandelholzgeruch vorhanden ist, ist dieser schwach und/oder von anderen Geruchsnoten überlagert.

Es war die Aufgabe der vorliegenden Erfindung, weitere Sandelholz-Riechstoffe anzugeben, wobei diese vorzugsweise positive Sekundäreigenschaften aufweisen sollten. Vorteilhafterweise sollten sich die anzugebenden Sandelholz-Riechstoffe durch ihre Stärke und Ausgiebigkeit ebenso auszeichnen wie durch eine hohe Diffusivität, so dass auch in geringen Dosierungen noch bemerkenswerte Effekte erzielt werden können.

Erfindungsgemäß wird diese Aufgabe gelöst durch eine Verbindung der Formel (Ia) oder (Ib) 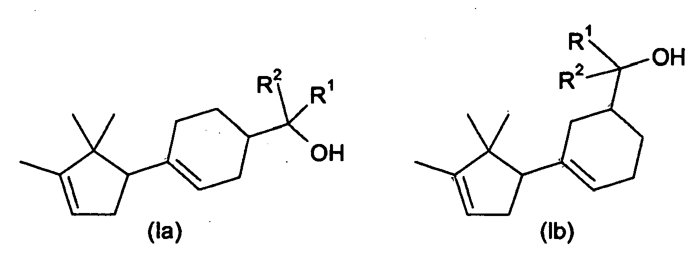 wobei in jeder der Formeln (la) und (Ib) R¹ und R² unabhängig voneinander H oder CH₃ sind.

Die erfindungsgemäße Verbindung kann dabei insbesondere in Form
(a) eines reinen optisch aktiven Enantiomers,
(b) einer racemischen Mischung verschiedener Enantiomere der diastereomeren Enantiomerenpaare, oder
(c) einer optisch aktiven Mischung verschiedener Enantiomere
vorliegen.

Sofern sich die Substituenten R¹ und R² voneinander unterscheiden, besitzen die Verbindungen der Formeln (la) und (Ib) drei chirale Zentren, so dass vier diastereomere Enantiomerenpaare existieren. Sind R¹ und R² in den Formeln (la) und (Ib) identisch, so gibt es nur zwei chirale Zentren und folglich auch nur zwei diastereomere Enantiomerenpaare.

Die erfindungsgemäßen Verbindungen der Formeln (la) und (Ib) besitzen einen parfümistisch interessanten Sandelholzgeruch und zeichnen sich überdies durch die gewünschten Sekundäreigenschaften (siehe oben) aus. Insbesondere besitzen die Verbindungen der Formeln (la) und (Ib) neben einer sehr intensiven Sandelholznote noch eine besonders hohe Diffusivität sowie eine enorme Substantivität. Dies ist angesichts der Offenbarung aus der US 5189013 überraschend, denn gemäß dieser Druckschrift besitzen Verbindungen mit einer Doppelbindung in Position 3 allenfalls einen sehr schwachen Sandelholzgeruch (vergleiche hierzu die letzten drei Einträge in der Tabelle I der US 5189013). Augenscheinlich ist die in den Verbindungen der Formeln (la) und (Ib) enthaltene Hydroxyalkyl-Funktion ―CHR¹R²OH verantwortlich dafür, dass im Unterschied zu den in der US 5189013 offenbarten Verbindungen mit einer Doppelbindung in Position 3 die erfindungsgemäßen Verbindungen einen ausgeprägten, typischen Sandelholzgeruch besitzen. Ein entsprechender Hinweis findet sich jedoch weder in der US 5189013 noch in Chemistry & Biodiversity, 1, 980-1021, 2004**.**

Sensorisch besonders wertvoll sind erfindungsgemäße Verbindungen der Formeln (la) und (Ib), für die gilt: R¹ ist CH₃ und R² ist H.

Außerordentlich wertvoll ist dabei die Verbindung der Formel (la) mit R¹ = CH₃ und R² = H. Diese Verbindung besitzt eine sehr intensive natürliche Sandelholznote, gepaart mit einer überraschend hohen Diffusivität und Substantivität (vergleiche hierzu Beispiel 1.3). Die entsprechende Verbindung der Formel (Ib) besitzt einen etwas weicheren Sandelholzgeruch.

Gemäß einem weiteren Aspekt betrifft die vorliegende Erfindung auch Mischungen, die aus zumindest einer erfindungsgemäßen Verbindung der Formel (la) und einer erfindungsgemäßen Verbindung der Formel (Ib) bestehen oder zumindest je eine dieser Verbindungen umfassen. In den erfindungsgemäßen Mischungen paart sich der sehr intensive natürliche Sandelholzgeruch der Verbindungen der Formel (Ia) mit der angenehmen Weichheit der Verbindungen der Formel (Ib) in einzigartiger Weise zu einem unverwechselbaren, komplexen und sehr intensiven Sandelholzgeruch. Hinsichtlich der Verwendung der bevorzugten Verbindungen der Formeln (la) bzw. (Ib) in einer erfindungsgemäßen Mischung gilt dabei das Vorgesagte entsprechend.

Wie weiter unten noch näher erläutert wird (vergleiche den Syntheseweg gemäß Schema I), lassen sich die erfindungsgemäßen Mischungen von Verbindungen der Formeln (Ia) und (Ib) in besonders wirtschaftlicher Weise synthetisieren.

Die erfindungsgemäßen Mischungen umfassen oder (vorzugsweise) bestehen aus einem oder mehreren Paaren von Verbindungen der Formeln (la) und (Ib), wobei in dem oder den Paaren die Substituenten R¹ und R² in der Verbindung der Formel (la) die gleiche Bedeutung haben wie in der Verbindung der Formel (Ib).

Besonders bevorzugt ist dabei eine erfindungsgemäße Mischung umfassend oder (vorzugsweise) bestehend aus einem Paar von Verbindungen der Formeln (la) und (Ib), wobei sowohl in der Verbindung der Formel (la) als auch in der Verbindung der Formel (Ib) R¹ CH₃ und R² H ist (vergleiche hierzu Beispiel 1, unten).

Die Erfindung betrifft auch die Verwendung einer erfindungsgemäßen Verbindung oder einer erfindungsgemäßen Mischung als Riechstoff, wobei hinsichtlich bevorzugter Verbindungen bzw. Mischungen das Vorgesagte entsprechend gilt.

Die Erfindung betrifft auch eine entsprechende Riechstoffmischung mit Sandelholzgeruch, umfassend eine erfindungsgemäße Verbindung oder eine erfindungsgemäße Mischung sowie vorzugsweise einen oder weitere (nichterfindungsgemäße) übliche Bestandteile wie Lösungsmittel, weitere Riechstoffe oder dergleichen. Hinsichtlich der bevorzugten Auswahl erfindungsgemäßer Verbindungen und Mischung gilt dabei selbstverständlich das zuvor Gesagte ensprechend.

Die Erfindung betrifft zudem parfümierte Produkte, die eine erfindungsgemäße Riechstoffmischung sowie einen Träger oder ein Substrat umfassen, der bzw. das in direktem Kontakt mit Riechstoffmischung steht. Das parfümierte Produkt kann dabei Vorteilhafterweise ausgewählt sein aus der Gruppe bestehend aus alkoholischen Parfüms, Körperpflegeprodukten und im Haushalt zu verwendenden Reinigungs- oder Pflegeprodukten.

Des Weiteren betrifft die Erfindung gemäß einem verwandten Aspekt ein Verfahren zum Erzeugen, Verstärken oder Modifizieren eines Sandelholzgeruchs in einer Mischung, mit folgenden Schritten:
- Bereitstellen einer erfindungsgemäßen Verbindung oder einer erfindungsgemäßen Mischung,
- Bereitstellen einer Komposition sonstiger Bestandteile und
- Vermischen der Komposition sonstiger Bestandteile mit einer Menge der erfindungsgemäßen Verbindung oder der erfindungsgemäßen Mischung, die ausreicht, (a) in der resultierenden Gesamtmischung einen Sandelholzgeruch zu erzeugen, (b) einen vorhandenen Sandelholzgeruch in der Komposition sonstiger Bestandteile zu verstärken oder (c) einen vorhandenen Sandelholzgeruch in der Komposition sonstiger Bestandteile zu modifizieren.

Die erfindungsgemäßen Verbindungen der Formel (Ia) und (Ib) können als Einzelstoffe oder in Form von Mischungen (siehe dazu auch oben) in einer Vielzahl von Riechstoffmischungen und parfümierten Produkten eingesetzt werden. Besonders vorteilhaft lassen sich die erfindungsgemäßen Verbindungen der oben angegebenen Formeln bzw. die erfindungsgemäßen Mischungen mit anderen Riechstoffen zu neuartigen Parfümkompositionen kombinieren.

Durch die Verwendung der erfindungsgemäßen Verbindungen bzw. Mischungen der oben angegebenen Formeln lassen sich bereits in geringer Dosierung in den resultierenden Parfümkompositionen (Riechstoffmischungen) Sandelholznoten erreichen, die sehr deutlich an Sandelholzöl erinnern, wobei der geruchliche Gesamteindruck auffallend harmonisiert, die Ausstrahlung wahrnehmbar erhöht und die Fixierung, d. h. das Haftvermögen der Parfümkomposition, deutlich verstärkt sind.

Beispiele für Riechstoffe, mit denen die erfindungsgemäßen Sandelholz Riechstoffe der Formel (la) und (Ib) vorteilhaft kombiniert werden können, finden sich z.B. in K. Bauer, D. Garbe und H. Surburg, Common Fragrance and Flavor Materials, 3rd. Ed., Wiley-VCH, Weinheim 1997.

Im einzelnen seien genannt:
Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos - Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptuscitriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon, bzw. daraus isolierten Inhaltsstoffen;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;
der aliphatischen Alkohole wie z. B. Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; der aliphatischen Aldehyde und deren 1,4-Dioxacycloalken-2-one wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Tridecensäurenitril; 2,12-Tridecensäurenitril; 3,7-Dimethyl-2,6-octadiensäurenitril; 3,7-Dimethyl-6-octen-säurenitril;
der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
der acyclischen Terpenalkohole wie z.B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon, alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);
der cyclischen Alkohole wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetra-methylcyclohexanon; 4-tert.-Pentylcyclohexanon; 5-Cyclohexadecen-1-on; 6,7-Dihydro-1,1,2,3,3-pentamethyl-4(5H)-indanon; 5-Cyclohexadecen-1-on; 8-Cyclo-hexadecen-1-on; 9-Cycloheptadecen-1-on; Cyclopentadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexen-carbaldehyd;
der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der aromatischen Kohlenwasserstoffe wie z. B. Styrol und Diphenylmethan;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B.; Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1 1 ,2,6-tetramethyl-3-(1-methyl-ethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentan-säurenitril; Methylanthranilat; Methy-N-methylanthranilat; Schiffsche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Parfümöle, welche die erfindungsgemäßen Verbindungen der oben genannten Formeln enthalten, können in flüssiger Form, unverdünnt oder mit einem Lösungsmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Parfümöle (Riechstoffmischungen), welche erfindungsgemäße Verbindungen enthalten, können an einem Trägerstoff absorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Parfümöle (Riechstoffmischungen), welche erfindungsgemäße Verbindungen enthalten, können auch mikroverkapselt, sprühgetrocknet, als Einschluss-Komplexe oder als Extrusions-Produkte vorliegen und in dieser Form dem zu parfümierenden Produkt hinzugefügt werden.

Gegebenenfalls können die Eigenschaften der derart modifizierten Parfümöle durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmaterialien z.B. aus Polyurethan-artigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erfolgen.

In Parfümkompositionen beträgt die eingesetzte Menge der erfindungsgemäßen Verbindungen vorzugsweise 0,05 bis 50 Gew.-%, vorzugsweise 0,5 bis 20 Gew.-%, bezogen auf das gesamte Parfümöl.

Parfümöle (Riechstoffmischungen), die erfindungsgemäße Verbindungen enthalten, können in konzentrierter Form, in Lösungen oder in oben beschriebener modifizierter Form verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

Die Erfindung betrifft auch ein Verfahren zur Herstellung einer erfindungsgemäßen Verbindung oder einer erfindungsgemäßen Mischung, mit folgenden Schritten:
- Bereitstellen oder Herstellen einer Verbindung der Formeln (IXa) oder (IXb) 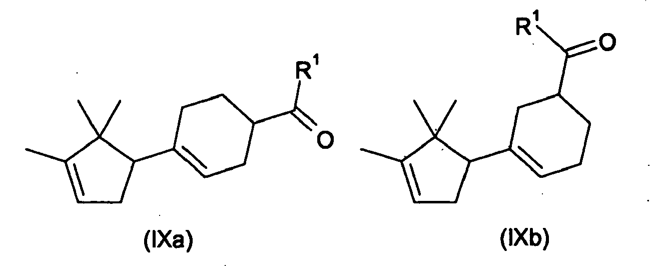 oder einer Mischung einer Verbindung der Formel (IXa) mit einer Verbindung der Formel (IXb),
wobei R¹ H oder CH₃ bedeutet,
- Reduzieren der Verbindung oder der Mischung, so dass eine erfindungsgemäße Verbindung bzw. Mischung mit R² gleich H gebildet wird, wobei R¹ die gleiche Bedeutung besitzt wie für die Verbindungen der Formeln (IXa) und (IXb) angegeben, bzw.
   nukleophile Addition einer metallorganischen Verbindung mit Methylanionencharakter an die Verbindung oder die in der Mischung enthaltenen Verbindungen, so dass eine erfindungsgemäße Verbindung bzw. Mischung mit R² gleich CH₃ gebildet wird, wobei R¹ die gleiche Bedeutung besitzt wie für die Verbindungen der Formeln (IXa) und (IXb) angegeben.

Die angegebenen Verbindungen der Formeln (IXa) und (IXb) sind dabei neu. Die Verbindungen der Formeln (IXa) und (IXb) oder die Mischungen einer Verbindung der Formel (IXa) mit einer Verbindung der Formel (IXb), wobei R¹ H oder CH₃ bedeutet, sind daher ebenfalls Gegenstand der vorliegenden Erfindung.

In dem erfindungsgemäßen Verfahren zur Herstellung der Verbindungen der Formeln (la) bzw. (Ib) bzw. der entsprechenden Mischungen mit R² = H erfolgt die Reduktion der Verbindung oder der Mischung (Formeln (IXa) und (IXb)) vorzugsweise mit einem Reduktionsmittel wie Natriumborhydrid oder Lithiumaluminiumhydrid.

Zur entsprechenden Herstellung von Verbindungen der Formeln (la) bzw. (Ib) bzw. der entsprechenden Mischungen mit R² = CH₃ erfolgt die nukleophile Addition vorzugsweise im Wege einer Grignard-Reaktion, zum Beispiel unter Verwendung eines Methylmagnesiumhalogenids wie Methylmagnesiumchlorid. Alternativ kann Methyllithium eingesetzt werden.

Diese erfindungsgemäßen Verfahren sind besonders geeignet zur Herstellung einer Mischung von Verbindungen der Formeln (la) und (Ib).

Zur Herstellung der (selbst ja neuen) Zwischenprodukte, das heißt der Verbindungen der Formeln (IXa) oder (IXb) oder (bevorzugt) einer Mischung einer Verbindung der Formel (IXa) mit einer Verbindung der Formel (IXb), wobei R¹ H oder CH₃ bedeutet, werden vorzugsweise die folgenden Schritte durchgeführt:
- Überführen einer Verbindung der Formel (X) 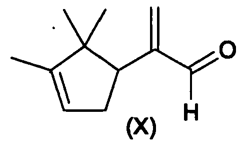 mittels Olefinierungs-Reaktion in eine Verbindung der Formel (XI), 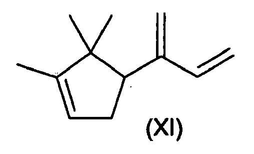
- Umsetzen der Verbindung der Formel (XI) mit einer Verbindung der Formel (XII) 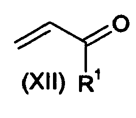 mittels Diels-Alder-Reaktion zu einer Mischung von Verbindungen der Formeln (IXa) und (IXb), wobei in Formel (XII) R¹ die oben (für (IXa), (IXb)) angegebene Bedeutung besitzt, sowie gegebenenfalls
- Trennen der Verbindungen der Formeln (IXa) und (IXb) voneinander.

Im ersten Reaktionsschritt wird beispielsweise α-Methylencampholenaldehyd, das heißt die Verbindung (X) in einer Wittig Reaktion mit Methyltriphenylphosphoniumbromid in Gegenwart von Kalium-*tert*-butanolat als Base umgesetzt (Fitjer, L., Quabeck, U., Synth. Commun., 1985, 15, 855-864). Dem Fachmann sind alternative Olefinierungs-Reaktionen zur Überführung der Verbindung der Formel (X) in die Verbindung der Formel (XI) bekannt.

Das als Ergebnis des ersten Reaktionsschrittes erhaltene Dien der Formel (XI) wird dann in einer Diels-Alder-Reaktion, typischerweise in Gegenwart von katalytischen Mengen Aluminiumtrichlorid, mit der α, β-ungesättigten Carbonylverbindung der Formel (XII) umgesetzt, in der R¹ die oben genannte Bedeutung hat.

Ausgehend von der Verbindung der Formel (X) lässt sich insgesamt in drei Schritten eine Verbindung bzw. Mischung der Formeln (la) und (Ib) herstellen. Das nachfolgende Schema 1 verdeutlicht die durchzuführenden Reaktionsschritte; die Angabe von Methyltriphenylphosphoniumbromid in Schema 1 ist dabei lediglich beispielhaft zu verstehen.

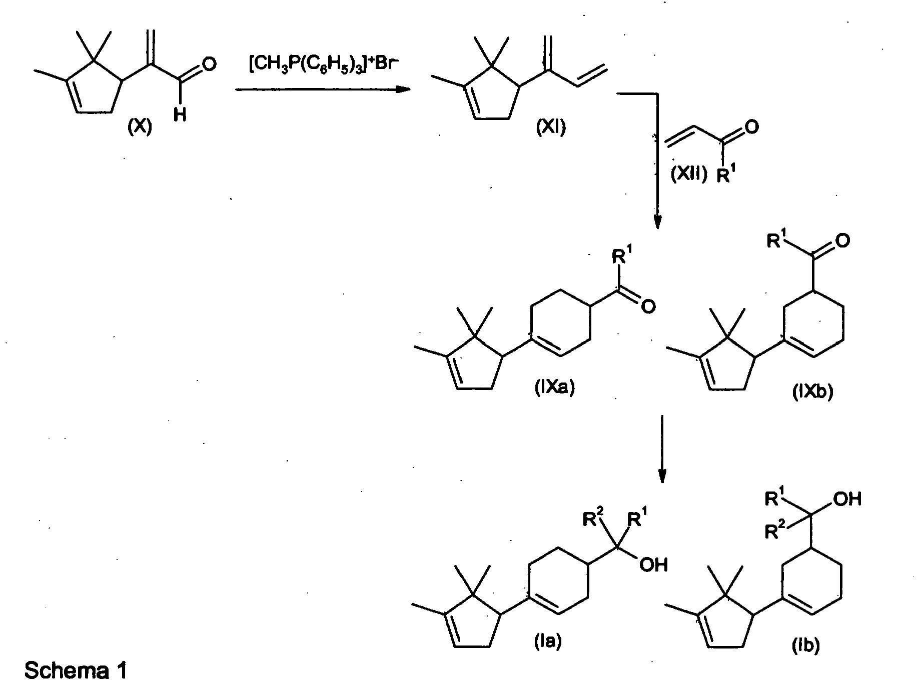

Die Erfindung betrifft auch ein (insoweit alternatives) Verfahren zur Herstellung von Verbindungen der Formel (la) 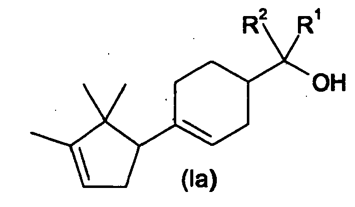 wobei R¹ H oder CH₃ und R² H bedeutet, mit folgenden Schritten:
- Bereitstellen oder Herstellen einer Verbindung der Formel (XIV), 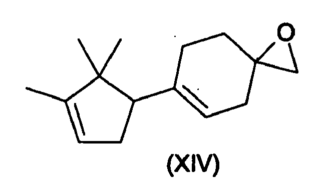
- Öffnen der Epoxidfunktion der Verbindung der Formel (XIV) und Bilden eines Aldehyds der Formel (IXa) mit R² = H 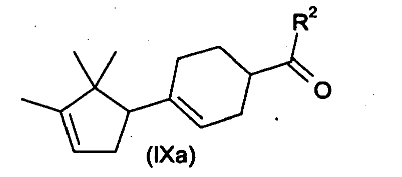
- Reduzieren des Aldehyds, so dass eine Verbindung der Formel (la) mit R¹, R² = H gebildet wird bzw.
   nukleophile Addition einer metallorganischen Verbindung mit Methylanionencharakter an den Aldehyd, so dass eine Verbindung der Formel (la) mit R¹ = CH₃, R² = H gebildet wird.

Die Verbindung (XIV), das heißt 6-(2,2,3-Trimethyl-cyclopent-3-enyl)-1-oxa-spiro[2.5]oct-5-en, ist selbst neu. Sie ist deshalb ebenfalls Gegenstand der vorliegenden Erfindung.

Die Öffnung der Epoxidfunktion der Verbindung der Formal (XIV) erfolgt vorzugsweise in Gegenwart von BF₃●OEt₂, was dann direkt zum Aldehyd (XIVa) führt. Dieser Aldehyd wird anschließend entweder mit einem Reduktionsmittel wie zum Beispiel Natriumborhydrid oder Lithiumaluminiumhydrid umgesetzt (zum Beispiel unter Standardbedingungen), so dass sich eine Verbindung der Formel (la) mit R¹, R² = H bildet, oder es wird die nukleophile Addition einer metallorganischen Verbindung mit Methylanioncharakter an den Aldehyd veranlasst, zum Beispiel im Wege einer Grignard-Reaktion (zum Beispiel unter Standardbedingungen) die Reaktion beispielsweise mit Methylmagnesiumhalogenid, vorzugsweise Methylmagnesiumchlorid, so dass eine Verbindung der Formel (Ia) mit R¹ = CH₃, R² = H gebildet wird.

Die erfindungsgemäße Verbindung der Formel (XIV) ist ein besonders geeignetes Zwischenprodukt zur Herstellung der Verbindungen der Formel (la) mit R² = H. Ein erfindungsgemäßes Verfahren zur Herstellung einer erfindungsgemäßen Verbindung der Formel (XIV) umfasst die folgenden Schritte:
- Bereitstellen oder Herstellen einer Verbindung der Formel (XIII), 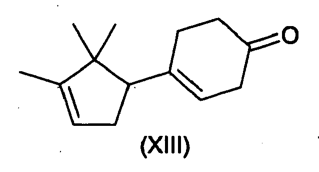
- Umwandeln der Carbonylfunktion der Verbindung der Formel (XIII) in eine Epoxidfunktion, so dass die Verbindung der Formel (XIV) resultiert.

Die Verbindung der Formel (XIII) wird dabei vorzugsweise durch Umsetzung mit Trimethylsulfoxoniumiodid, vorzugsweise in Gegenwart von Natriumhydrid, in das Epoxid der Formel (XIV) überführt, vergleiche E. J. Corey et al., J. Am. Chem. Soc., 87, 1353-1364, 1965.

Ausgehend von der Verbindung der Formel (XIII) lässt sich in insgesamt drei Schritten eine Verbindung der Formel (la) mit R² = H herstellen. Das nachfolgende Schema 2 verdeutlicht die durchzuführenden Reaktionsschritte. Die Angabe von BF₃•OEt₂ in Schema 2 ist dabei zwar lediglich beispielhaft zu verstehen, dennoch ist die entsprechende Verfahrensgestaltung bevorzugt. Vergleiche zur Umlagerung des Epoxids in Gegenwart von BF₃•OEt₂ zum Aldehyd (IXa) Y. Kita et al., Tetrahedron, 55, 4979-4998, 1999.

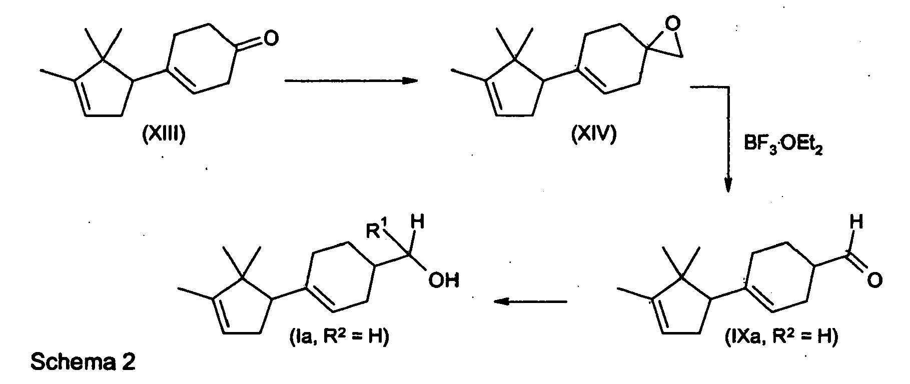

Die folgenden, nicht limitierenden Beispiele erläutern die Erfindung.

### Beispiel 1: 1 -[4-(2,2,3-Trimethyl-cyclopent-3-enyl)-cyclohex-3-enyl]-ethanol (la mit R¹ = -CH₃ und R² = H)/1-[3-(2,2,3-Trimethyl-cyclopent-3-enyl)-cyclohex-3-enyl]-ethanol (Ib mit R¹ = -CH₃ und R² = H)

### 1.1. 1,5,5-Trimethyl-4-(1-methylen-allyl)-cyclopenten (XI):

Zu einer Lösung aus Methyltriphenylphosphoniumbromid (708.1 g, 1.98 mol) in Diethylether (1900 ml) gibt man Kalium-*tert*-butanolat (222.5 g, 1.98 mol) hinzu. Die sich bildende gelbe Lösung wird nach beendeter Zugabe auf Rückflusstemperatur erhitzt und nach 60 Minuten tropft man α-Methylencampholenaldehyd (X, 247.4 g, 1.51 mol) hinzu. Nach beendeter Reaktion lässt man auf Raumtemperatur abkühlen und gibt unter starkem rühren Pentan (600 ml) und Wasser (600 ml) hinzu. Der entstandene Niederschlag wird abgesaugt, anschließend trennt man die Phasen, und extrahiert die wässrige Phase noch dreimal mit Diethylether (je 750 ml). Die vereinigten organischen Phasen werden getrocknet, abfiltriert und einrotiert. Das erhaltene Rohprodukt wird nochmals in Pentan (500 ml) aufgenommen und der sich bildende Niederschlag wird abgesaugt. Anschließend entfernt man das Lösungsmittel am Rotationsverdampfer und erhält 281.6 g Rohprodukt (XI), mit einem Produktgehalt von 79%. Das erhaltene Rohprodukt wird ohne weitere Reinigung in die nächste Stufe eingesetzt. Zur spektroskopischen Strukturbestimmung wird eine kleine Probe des Rohproduktes flashchromatographisch gereinigt.

¹H-NMR (400 MHz, CDCl₃): δ (ppm) = 0.75 (s, 3H), 1.07 (s, 3H), 1.61 (td, J = 2.1, 1.6 Hz, 3H), 2.33 (dquin, J = 8.2, 2.1 Hz, 2H), 2.90 (t, J = 8.2 Hz, 1 H), 4.98-5.06 (m, 2H), 5.20-5.23 (m, 1H), 5.27-5.33 (m, 1H), 5.33 (dd, J = 17.4, 1.3 Hz, 1H), 6.38 (ddd, J = 17.4, 10.8, 0.9 Hz, 1 H).

¹³C-NMR (100 MHz, CDCl₃): δ (ppm) = 12.8, 21.4, 27.0, 34.9, 47.8, 51.0, 113.0, 114.9, 121.5, 140.4, 147.2, 147.3.

### 1.2 1-[4-(2,2,3-Trimethyl-cyclopent-3-enyl)-cyclohex-3-enyl]-ethanon (IXa mit R¹ = -CH₃)/ 1-[3-(2,2,3-Trimethyl-cyclopent-3-enyl)-cyclohex-3-enyl]-ethanon (IXb mit R¹ = -CH₃):

Zu einer heterogenen Lösung von Aluminiumchlorid (23.79g, 0.18 mol) in Toluol (350 ml) tropft man Methylvinylketon (124.8 g, 1.78 mol), gelöst in Toluol (75 ml), so zu, dass die Temperatur 25°C nicht übersteigt. Man lässt 30 Minuten rühren, bevor man das Dien (XI, 264.0 g, 1.28 mol, 79% Reinheit), gelöst in Toluol (75 ml), zugibt. Jetzt erhitzt man für 24 Stunden auf 90°C. Nach beendeter Reaktion gießt man den Reaktionsansatz auf gesättigte NaHCO₃-Lösung (500 ml), trennt die Phasen und wäscht die organische Phase mit gesättigter NaHCO₃-Lösung bis zur Neutralität. Anschließend wird die organische Phase über Na₂SO₄ getrocknet, abfiltriert und einrotiert. Man erhält 404.1 g Rohprodukt, wobei die beiden Isomeren (IXa) und (IXb) mit R¹ = -CH₃ im Verhältnis 3:1 und in einer Gesamtreinheit von 73.2% entstehen. Das erhaltene Rohprodukt wird ohne weitere Reinigung in die nächste Stufe eingesetzt. Zur spektroskopischen Strukturbestimmung wird eine kleine Probe des Rohproduktes flashchromatographisch (Cyclohexan/EtOAc = 10:1, R_{f} = 0.24) gereinigt.

Die spektroskopischen Daten beziehen sich auf (IXa mit R¹ = -CH₃).

¹H-NMR (400 MHz, CDCl₃): δ (ppm) = 0.73 (s, 3H), 1.04 (s, 3H), 1.56-1.60 (m, 3H), 1.89-2.27 (m, 7H), 2.17 (s, 3H), 2.23-2.37 (m, 1 H), 2.39-2.46 (m, 1 H), 2.51-2.60 (m, 1 H), 5.22-5.27 (m, 1 H), 5.45-5.52 (m, 1 H).

¹³C-NMR (100 MHz, CDCl₃): δ (ppm) = 12.7, 20.6, 25.1, 26.7, 27.2, 27.9, 28.7, 32.4, 47.5, 48.1, 57.3, 120.5, 121.2, 137.5, 146.9, 211.2.

Geruch: Schwache Sandelholznote, holzig-süß, moosig, nussig.

### 1.3 1-[4-(2,2,3-Trimethyl-cyclopent-3-enyl)-cyclohex-3-enyl]-ethanol (Ia mit R¹ = -CH₃ und R² = H)/ 1-[3-(2,2,3-Trimethyl-cyclopent-3-enyl)-cyclohex-3-enyl]-ethanol (Ib mit R¹ = -CH₃ und R² = H):

Zu einer auf 0°C abgekühlten Lösung des Reaktionsgemisches nach Vorschrift 1.2 (IXa/IXb = 3:1, 253.6 g, 0.80 mol, Reinheit 73.2%) in Ethanol (600 ml) gibt man portionsweise Natriumborhydrid (15.1 g, 0.4 mol). Nach beendeter Zugabe lässt man auf Raumtemperatur kommen und rührt eine weitere Stunde. Nach beendeter Reaktion gibt man 2M HCl zu, bis der Reaktionsansatz pH = 7 erreicht. Anschließend rotiert man das Ethanol ab und nimmt den Rückstand in gesättigter NaCl-Lösung (300 ml) auf. Jetzt wird dreimal mit Diethylether (je 500 ml) extrahiert, bevor man die vereinigten organischen Phasen über Na₂SO₄ trocknet, abfiltriert und einrotiert. Man erhält 247.1 g Rohprodukt, wobei die beiden Isomere (la) und (Ib) mit R¹ = -CH₃ und R² = H im Verhältnis 3:1 und in einer Gesamtreinheit von 76.5% entstehen. Anschließende Vakuumdestillation an einer 30 cm Füllkörperkolonne (Sdp.: 100-103°C, 0.11 mbar) liefert den gewünschten Sandelholzriechstoff (Ia/Ib = 3:1, mit R¹ = -CH₃ und R² = H) in 97%-iger Reinheit.

Die beiden Regioisomere (la) und (Ib) mit R¹ = -CH₃ und R² = H konnten durch präparative HPLC (Säule: GROM Saphir 110 Si, 5µm, 125x20 mm; Eluent: Methanol/Wasser = 3:1; Flussrate: 25 ml/min; Druck: 120 bar) getrennt, analysiert, spektroskopisch vermessen und geruchlich evaluiert werden.

1-[4-(2,2,3-Trimethyl-cyclopent-3-enyl)-cyclohex-3-enyl]-ethanol (la mit R¹ = -CH₃ und R² = H)

¹H-NMR (400 MHz, CDCl₃): δ (ppm) = 0.73 (s, 3H), 1.04 (s, 3H), 1.19 (d, J = 6.3 Hz, 3H), 1.25-1.38 (m, 1 H), 1.54 (s, 1 H, OH), 1.43-1.60 (m, 1 H), 1.59 (td, J = 2.4, 1.6 Hz, 3H), 1.68-2.12 (m, 5H), 2.12-2.22 (m, 1 H), 2.25-2.36 (m, 1 H), 2.43 (t, J = 8.4 Hz, 1 H), 3.57 (quint, J = 6.4 Hz, 1 H), 5.24-5.27 (m, 1 H), 5.45-5.52 (m, 1 H).

¹³C-NMR (100 MHz, CDCl₃): δ (ppm) = 12.8, 20.7, 20.8, 25.1, 26.8, 28.1, 29.5, 32.5, 41.5, 48.2, 57.6, 71.9, 121.5, 121.6, 137.8, 147.4.

Geruch: sehr intensive Sandelholznote, natürlich an β-Santalol erinnernd, etwas milchig-fettig mit schwachem Moschuscharakter, enorme Raumwirkung (Diffusivität) und Haftung (Substantivität).

1-[3-(2,2,3-Trimethyl-cyclopent-3-enyl)-cyclohex-3-enyl]-ethanol (Ib mit R¹ = -CH₃ und R² = H)

¹H-NMR (400 MHz, CDCl₃): δ (ppm) = 0.74 (s, 3H), 1.05 (s, 3H), 1.20 (d, J = 6.3 Hz, 3H), 1.25-1.38 (m, 1 H), 1.46-1.58 (m, 1 H), 1.58-1.61 (m, 3H), 1.64-1.76 (m, 1H), 1.78-.96 (m, 2H), 2.02-2.22 (m, 4H), 2.26-2.38 (m, 4H), 2.40-2.48 (m, 1H), 3.61 (quint, J = 6.4 Hz, 1 H), 5.24-5.28 (m, 1 H), 5.46-5.53 (m, 1 H).

¹³C-NMR (100 MHz, CDCl₃): δ (ppm) = 12.8, 20.7, 20.8, 24.4, 25.6, 26.9, 32.1, 32.5, 41.8, 48.2, 57.8, 71.7, 121.6, 122.5, 137.0, 147.4.

Geruch: Weiche Sandelholznote, trocken, etwas staubig.

Der Geruch der Isomerenmischung (la) und (lb) mit R¹ = -CH₃ und R² = H im Verhältnis 3:1, wird wie folgt beschrieben: Sehr intensive weiche Sandelholzote, an β-Santalol erinnernde Natürlichkeit, mit enormer Raumwirkung (Diffusivität) und Haftung (Substantivität).

### Beispiel 2: 2-[4-(2,2,3-Trimethyl-cyclopent-3-enyl)-cyclohex-3-enyl]-propan-2-ol (Ia mit R¹ und R² = -CH₃)/ 2-[3-(2,2,3-Trimethyl-cyclopent-3-enyl)-cyclohex-3-enyl]-propan-2-ol (Ib mit R¹ und R² = -CH₃)

Zu einer 3M Methylmagnesiumchlorid-Lösung in THF (15 ml, 45 mmol) tropft man (IXa)/(IXb) = 3:1 mit R¹ = -CH₃ (8.21 g, 35 mmol), gelöst in Diethylether (10 ml), so zu, dass die Temperatur 35°C nicht übersteigt. Nach beendetem zutropfen rührt man noch 1 Stunde bei Raumtemperatur. Nach beendeter Reaktion gießt man die Reaktionslösung auf kalte NH₄Cl-Lösung (15 ml), trennt die Phasen und extrahiert die wässrige Phase noch zweimal mit Diethylether (je 50 ml). Die vereinigten organischen Phasen werden noch je einmal mit gesättigter NaHCO₃-Lösung und gesättigter NaCl-Lösung gewaschen, bevor sie über Na₂SO₄ getrocknet, abfiltriert und einrotiert werden. Man erhält 9.52 g Rohprodukt, welches durch Flashchromatographie (Cyclohexan/EtOAc = 10:1, R_{f} = 0.21) gereinigt wird. Man erhält (Ia)/(Ib) mit R¹ und R² = -CH₃ im Verhältnis 3:1.

Die spektroskopischen Daten beziehen sich auf (la mit R¹ und R² = -CH₃).

¹H-NMR (400 MHz, CDCl₃): δ (ppm) = 0.75 (s, 3H), 1.06 (s, 3H), 1.18 (s, 3H), 1.20 (s, 3H), 1.18-1.30 (m, 1 H), 1.47-1.60 (m, 1 H), 1.60 (td, J = 2.3, 1.7 Hz, 3H), 1.80-2.05 (m, 3H), 2.05-2.23 (m, 4H), 2.23-2.38 (m, 1 H), 2.43 (t, J = 8.2 Hz, 1 H), 5.23-5.28 (m, 1 H), 5.46-5.53 (m, 1 H).

¹³C-NMR (100 MHz, CDCl₃): δ (ppm) = 12.7, 20.6, 24.1, 26.1, 26.8, 27.0, 27.1, 27.3, 30.4, 32.4, 45.2, 57.4, 72.5, 121.3, 121.6, 137.5, 147.1.

Geruch: Sandelholznote, etwas fettig.

### Beispiel 3: [4-(2,2,3-Trimethyl-cyclopent-3-enyl)-cyclohex-3-enyl]-methanol (Ia mit R¹ und R² = H)/ [3-(2,2,3-Trimethyl-cyclopent-3-enyl)-cyclohex-3-enyl]-methanol (Ib mit R¹ und R² = H)

### 3.1 4-(2,2,3-Trimethyl-cyclopent-3-enyl)-cyclohex-3-encarbaldehyd (IXa mit R¹ = H)/ 3-(2,2,3-Trimethyl-cyclopent-3-enyl)-cyclohex-3-encarbaldehyd (IXb mit R¹ = H):

Zu einer heterogenen Lösung von Aluminiumchlorid (12.11 g, 91.00 mmol) in Toluol (700 ml) tropft man Acrolein (47.85 g, 0.85 mol), gelöst in Toluol (275 ml), so zu, dass die Temperatur 25°C nicht übersteigt. Man lässt 30 Minuten rühren, bevor man das Dien (XI, 143.8 g, 0.70 mol, 79% Reinheit), gelöst in Toluol (45 ml), zugibt. Jetzt erhitzt man für 24 Stunden auf 90°C. Nach beendeter Reaktion gießt man den Reaktionsansatz auf gesättigte NaHCO₃-Lösung (300 ml), trennt die Phasen und wäscht die organische Phase mit gesättigter NaHCO₃-Lösung bis zur Neutralität. Anschließend wird die organische Phase über Na₂SO₄ getrocknet, abfiltriert und einrotiert. Man erhält 195.4 g Rohprodukt, wobei die beiden Isomeren (IXa) und (IXb) mit R¹ = H im Verhältnis 3:1 und in einer Gesamtreinheit von 66.5% entstehen. Das erhaltene Rohprodukt wird ohne weitere Reinigung in die nächste Stufe eingesetzt. Zur spektroskopischen Strukturbestimmung wird eine kleine Probe des Rohproduktes flashchromatographisch (Cyclohexan/EtOAc = 10:1, R_{f} = 0.22) gereinigt.

Die Massenspektroskopischen Daten beziehen sich auf (IXa mit R¹ = H).

MS: m/z (%) = 41 (C₃H₅⁺, 82), 55 (C₄H₇⁺, 43), 67 (C₅H₇⁺, 43), 79 (C₆H₇⁺, 75), 91 (C₇H₇⁺, 100), 105 (C₈H₉⁺, 67), 119 (C₉H₁₁⁺, 60), 148 (M⁺ - C₄H₆O, 56), 175 (M⁺ - C₃H₇⁺, 73), 203 (M⁺ - CH₃), 218 (M⁺, 35).

Geruch: Intensive Sandelholznote, schöne Natürlichkeit.

### 3.2 [4-(2,2,3-Trimethyl-cyclopent-3-enyl)-cyclohex-3-enyl]-methanol (la mit R¹ und R² = H)/ [3-(2,2,3-Trimethyl-cyclopent-3-enyl)-cyclohex-3-enyl]-methanol (Ib mit R¹ und R² = H):

Zu einer Suspension aus Lithiumaluminiumhydrid (10.16 g, 0.27 mol) in Diethylether (400 ml) tropft man den Aldehyd (Ia/Ib = 3:1, mit R¹ und R² = H, 178.0 g, 0.54 mol, 66.5% Reinheit), gelöst in Diethylether (150 ml), so zu, dass der Diethylether leicht siedet. Nach beendeter Zugabe lässt man noch 30 Minuten rühren, bevor man nacheinander n-Hexan (150 ml), Wasser (150 ml) und 15%ige NaOH (100 ml) zugibt. Der enstandene Niederschlag wird abgesaugt, die Phasen werden getrennt und die wässrige Phase wird noch dreimal mit Diethylether (je 250 ml) extrahiert. Die vereinigten organischen Phasen werden über Na₂SO₄ getrocknet, abfiltriert und einrotiert. Man erhält 151.8 g Rohprodukt in 68%-iger Reinheit. Anschließende Vakuumdestillation an einer 30 cm Füllkörperkolonne (Sdp.: 92-94°C, 0.07 mbar) liefert den gewünschten Sandelholzriechstoff (Ia/Ib = 3:1, mit R¹ und R² = H) in 98%-iger Reinheit.

Die spektroskopischen Daten beziehen sich auf (la mit R¹ und R² = H).

¹H-NMR (400 MHz, CDCl₃): δ (ppm) = 0.74 (s, 3H), 1.05 (s, 3H), 1.18-1.38 (m, 2H), 1.59 (td, J = 2.2, 1.6 Hz, 3H), 1.72-1.86 (m, 3H), 1.91-2.05 (m, 1 H), 2.06-2.22 (m, 3H), 2.25-2.37 (m, 1 H), 2.43 (t, J = 8.3 Hz, 1 H), 3.49-3.59 (m, 2H), 5.24-5.28 (m, 1 H), 5.46-5.50 (m, 1 H).

¹³C-NMR (100 MHz, CDCl₃): δ (ppm) = 12.7, 20.7, 25.9,26.9, 27.7, 28.9, 32.5, 36.6, 48.2, 57.6, 67.8, 121.5, 121.6, 138.1, 147.3.

Geruch: Schöne Sandelholznote mit Moschusaspekten.

### Beispiel 4: 1-[4-(2,2,3-Trimethyl-cyclopent-3-enyl)-cyclohex-3-enyl]-ethanol (Ia mit R¹ = -CH₃ und R² = H)

### 4.1 6-(2,2,3-Trimethyl-cyclopent-3-enyl)-1-oxa-spiro[2.5]oct-5-en (XIV):

Zu einer Suspension aus Natriumhydrid (60% in Mineralöl, 1.00 g, 25.54 mmol) in DMSO (10 ml) gibt man portionsweise Trimethylsulfoxoniumiodid (4.78 g, 21.63 mmol). Jetzt lässt man 30 Minuten rühren, bevor man 4-(2,2,3-Trimethyl-cyclopent-3-enyl)-cyclohex-3-enon (XIII, siehe US 5189013, 2.78 g, 13.64 mmol), gelöst in DMSO (10 ml), zugibt. Nach beendeter Zugabe lässt man weitere 30 Minuten rühren und gießt anschließend die Reaktionslösung auf Eis (25 ml). Jetzt verdünnt man mit Diethylether (50 ml), trennt die Phasen und extrahiert die wässrige Phase noch dreimal mit Diethylether (je 50 ml). Die vereinigten organischen Phasen werden noch einmal mit gesättigter NaCl-Lösung gewaschen, bevor sie über Na₂SO₄ getrocknet, abfiltriert und einrotiert werden. Man erhält 3.08 g Rohprodukt, welches anschließend flashchromatographisch (Cyclohexan/EtOAc = 20:1, R_{f} = 0.22) gereinigt wird. Man erhält 2.21 g eines farblosen Öls.

¹H-NMR (400 MHz, CDCl₃): δ (ppm) = 0.78 (s, 3H), 1.06 (s, 3H), 1.51-1.59 (m, 1 H), 1.60 (td, J = 2.4, 1.7 Hz, 3H), 1.72-1.84 (m, 1 H), 1.98-2.54 (m, 7H), 2.68-2.72 (m, 2H), 5.25-5.28 (m, 1 H), 5.47-5.51 (m, 1 H).

¹³C-NMR (100 MHz, CDCl₃): δ (ppm) = 12.7, 20.9, 26.8, 27.6, 29.7, 32.8, 33.0, 48.3, 53.9, 57.2, 57.5, 120.4, 121.5, 138.5, 147.3.

### 4.2 4-(2,2,3-Trimethyl-cyclopent-3-enyl)-cyclohex-3-encarbaldehyd (IXa mit R² = H):

Zu einer Lösung von BF₃·OEt₂ (1.7 ml) in Toluol (20 ml) werden 6-(2,2,3-Trimethyl-cyclopent-3-enyl)-1-oxa-spiro[2.5]oct-5-en (XIV, 2.72 g, 12.5 mmol), gelöst in Toluol (5 ml), getropft. Nach 2 Stunden gießt man die Reaktionslösung auf Eis (10 ml), trennt anschließend die Phasen und wäscht die organische Phase noch einmal mit gesättigter NaCl-Lösung. Die organische Phase wird nun über Na₂SO₄ getrocknet, abfiltriert und einrotiert. Man erhält 2.07 g Rohprodukt mit einer Reinheit von 83%, welches ohne weitere Reinigung in die nächste Stufe eingesetzt wird.

Die Massenspektroskopischen Daten entsprechen denen aus Beispiel 3.1.

Geruch: Sehr intensive, natürliche Sandelholznote.

### 4.3 1-[4-(2,2,3-Trimethyl-cyclopent-3-enyl)-cyclohex-3-enyl]-ethanol (la mit R¹ = -CH₃ und R² = H):

Zu einer 3M Methylmagnesiumchlorid-Lösung in THF (3.33 ml, 10.00 mmol) tropft man (IXa) mit R² = H (2.00 g, 7.60 mmol), gelöst in Diethylether (10 ml), so zu, dass die Temperatur 35°C nicht übersteigt. Nach beendetem Zutropfen rührt man noch 1 Stunde bei Raumtemperatur. Nach beendeter Reaktion gießt man die Reaktionslösung auf kalte NH₄Cl-Lösung (10 ml), trennt die Phasen und extrahiert die wässrige Phase noch zweimal mit Diethylether (je 20 ml). Die vereinigten organischen Phasen werden noch je einmal mit gesättigter NaHCO₃-Lösung und gesättigter NaCl-Lösung gewaschen, bevor sie über Na₂SO₄ getrocknet, abfiltriert und einrotiert werden. Man erhält 1.95 g Rohprodukt, welches durch Flashchromatographie (Cyclohexan/EtOAc = 25:1, R_{f} = 0.22) gereinigt wird, so dass man (la) mit R¹ = -CH₃ und R² = H erhält.

Die spektroskopischen Daten, wie auch der Geruch entsprechen denen aus Beispiel 1.3, für (la) mit R¹ = -CH₃ und R² = H.

### Beispiel 5: Parfümöl-Komposition

Das nachfolgend angegebene Parfümöl kann zur Parfümierung diverser kosmetischer Produkte, insbesondere für Schaumbad und Shampoo verwendet werden.

### Zusammensetzung:

| Ingredienzien | Gewichtsteile |
|---|---|
| 1. Vertocitral 10% in DPG | 6.0 |
| | |
| 2. Bergamottöl echt | 15.0 |
| | |
| 3. Linalylacetat | 30.0 |
| | |
| 4. Citral Nat. | 0.3 |
| | |
| 5. Aldehyd C14 sog. | 1.0 |
| | |
| 6. Decalacton gamma | 1.2 |
| | |
| 7. Linalool | 18.0 |
| | |
| 8. Citronellol 950 | 0.6 |
| | |
| 9. Geraniol supra. | 0.6 |
| | |
| 10. Geranylacetat Pure | 0.3 |
| | |
| 11. Nerylacetat 10% in DPG | 4.0 |
| | |
| 12. Damascon Alpha | 0.4 |
| | |
| 13. Hedion HC/30 | 280.0 |
| | |
| 14. Veloutone 10% in DPG | 1.0 |
| 15. Isoeugenolacetat | 2.0 |
| | |
| 16. Heliotropin | 26.0 |
| | |
| 17. Ethylvanillin | 0.9 |
| | |
| 18. Vanillin | 17.0 |
| | |
| 19. Iso E Super | 140.0 |
| | |
| 20. Sandranol | 70.0 |
| | |
| 21. Teak Base | 50.0 |
| | |
| 22. Cetalox | 0.6 |
| | |
| 23. Ethylenbrassylat | 22.0 |
| | |
| 24. Globalide | 55.0 |
| | |
| 25. Galaxolid 50% in IPM | 140.0 |
| | |
| 26. Dipropylenglycol | 48.1 |

| | |
|---|---|
| DPG = Dipropylenglycol; IPM = Isopropylmyristat | |

Der Zusatz von 70 Gewichtsteilen [1-[4-(2,2,3-Trimethyl-cyclopent-3-enyl)-cyclohex-3-enyl]-ethanol/1-[3-(2,2,3-Trimethyl-cyclopent-3-enyl)-cyclohex-3-enyl]-ethanol im Verhältnis - 3:1 führt zu einer deutlich wahrnehmbaren Harmonisierung der blumig-vanilligen Herznote. Darüber hinaus verleiht die intensive weiche, an β-Santalol erinnernde Sandelholzote, der vorliegenden Komposition eine hervorragende Strahlung, gepaart mit einer enormen Raumwirkung und gesteigerten Haftung. Hierbei setzt sich besonders der wertvolle Charakter von [1-[4-(2,2,3-Trimethyl-cyclopent-3-enyl)-cyclohex-3-enyl]-ethanol/1-[3-(2,2,3-Trimethyl-cyclopent-3-enyl)-cyclohex-3-enyl]-ethanol im Ver― hältnis - 3:1 durch.

## Patentansprüche

1. Verbindung der Formel (Ia) oder (Ib) 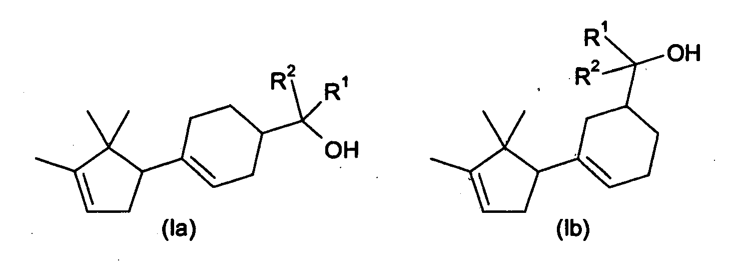 wobei in jeder der Formeln (Ia) und (Ib) R¹ und R² unabhängig voneinander H oder CH₃ sind.

2. Verbindung nach Anspruch 1, wobei die Verbindung in Form
(a) eines reinen optisch aktiven Enantiomers,
(b) einer racemischen Mischung verschiedener Enantiomere der diastereomeren Enantiomerenpaare, oder
(c) einer optisch aktiven Mischung verschiedener Enantiomere vorliegt.

3. Verbindung nach Anspruch 1 oder 2, wobei gilt:
R¹ ist CH₃
und
R² ist H.

4. Mischung umfassend oder bestehend aus zumindest je einer Verbindung der Formel (Ia) nach einem der vorangehenden Ansprüche und der Formel (Ib) nach einem der vorangehenden Ansprüche.

5. Mischung nach Anspruch 4, umfassend oder bestehend aus ein oder mehreren Paaren von Verbindungen der Formeln (Ia) und (Ib), wobei in dem oder den Paaren die Substituenten R¹ und R² in der Verbindung der Formel (la) die gleiche Bedeutung haben wie in der Verbindung der Formel (Ib).

6. Mischung nach Anspruch 5, umfassend oder bestehend aus einem Paar von Verbindungen der Formeln (Ia) und (Ib), wobei sowohl in der Verbindung der Formel (Ia) als auch in der Verbindung der Formel (Ib)
R¹ CH₃
und
R² H ist.

7. Verwendung einer Verbindung nach einem der Ansprüche 1 bis 3 oder einer Mischung nach einem der Ansprüche 4 bis 6 als Riechstoff.

8. Riechstoffmischung mit Sandelholzgeruch, umfassend eine Verbindung nach einem der Ansprüche 1 bis 3 oder eine Mischung nach einem der Ansprüche 4 bis 6 sowie vorzugsweise einen oder weitere übliche Bestandteile.

9. Parfümiertes Produkt, umfassend
- eine Riechstoffmischung nach Anspruch 8 sowie
- einen Träger oder ein Substrat, der bzw. das in direktem Kontakt mit der Riechstoffmischung steht.

10. Verfahren zur Herstellung einer Verbindung nach einem der Ansprüche 1 bis 3 oder einer Mischung nach einem der Ansprüche 4 bis 6, mit folgenden Schritten:
- Bereitstellen oder Herstellen einer Verbindung der Formeln (IXa) oder (IXb) 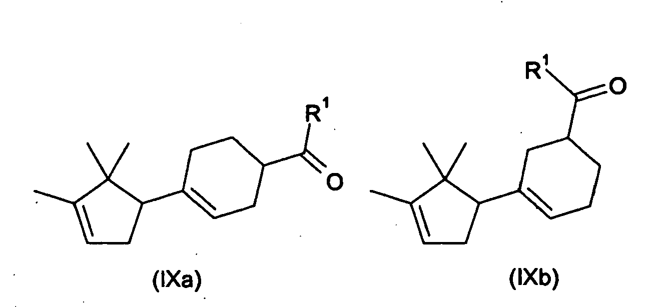 oder einer Mischung einer Verbindung der Formel (IXa) mit einer Verbindung der Formel (IXb),
wobei, R¹ H oder CH₃ bedeutet,
- Reduzieren der Verbindung oder der Mischung, so dass eine Verbindung nach einem der Ansprüche 1 bis 3 bzw. eine Mischung nach einem der Ansprüche 4 bis 6 mit R² gleich H gebildet wird, wobei R¹ die gleiche Bedeutung besitzt wie für die Verbindungen der Formeln (IXa) und (IXb) angegeben, bzw. nukleophile Addition einer metallorganischen Verbindung mit Methylanionencharakter an die Verbindung oder die in der Mischung enthaltenen Verbindungen, so dass eine Verbindung nach einem der Ansprüche 1 bis 3 bzw. eine Mischung nach einem der Ansprüche 4 bis 6 mit R² gleich CH₃ gebildet wird, wobei R¹ die gleiche Bedeutung besitzt wie für die Verbindungen der Formeln (IXa) und (IXb) angegeben.

11. Verbindung der Formel (IXa) oder (IXb) 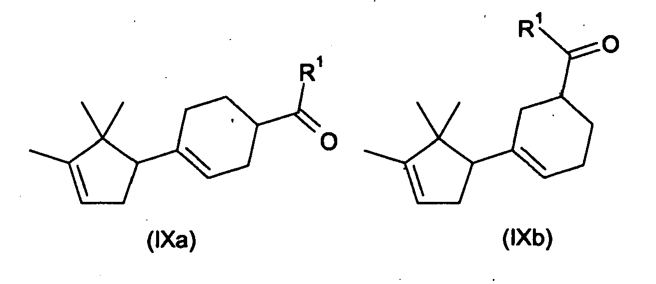 oder Mischung einer Verbindung der Formel (IXa) mit einer Verbindung der Formel (IXb),
wobei R¹ H oder CH₃ bedeutet.

12. Verfahren zur Herstellung einer Verbindung der Formeln (IXa) oder (IXb) nach Anspruch 11 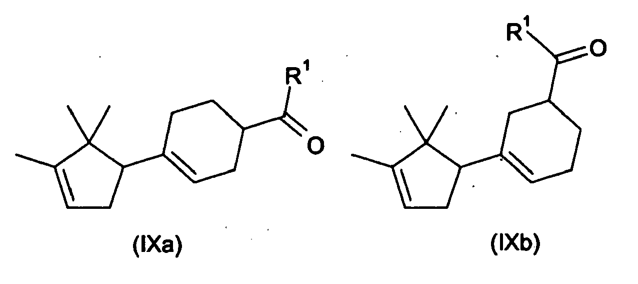 oder einer Mischung einer Verbindung der Formel (IXa) mit einer Verbindung der Formel (IXb),
wobei R¹ H oder CH₃ bedeutet,
mit folgenden Schritten:
- Überführen einer Verbindung der Formel (X) 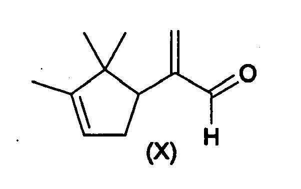 mittels Olefinierungs-Reaktion in eine Verbindung der Formel (XI), 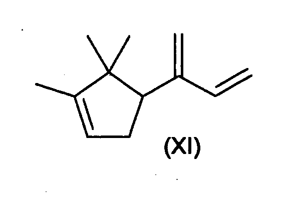
- Umsetzen der Verbindung der Formel (XI) mit einer Verbindung der Formel (XII) 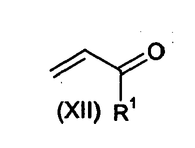 mittels Diels-Alder-Reaktion zu einer Mischung von Verbindungen der Formeln (IXa) und (IXb), wobei in Formel (XII) R¹ die oben angegebene Bedeutung besitzt,
sowie gegebenenfalls
- Trennen der Verbindungen der Formeln (IXa) und (IXb) voneinander.

13. Verfahren zur Herstellung einer Verbindung der Formel (Ia) nach Anspruch 1 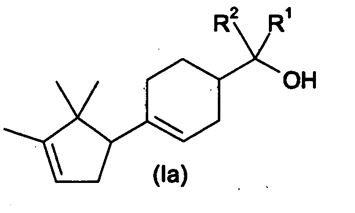 wobei
R¹ H oder CH₃
und
R² H bedeutet,
mit folgenden Schritten:
- Bereitstellen oder Herstellen einer Verbindung der Formel (XIV), 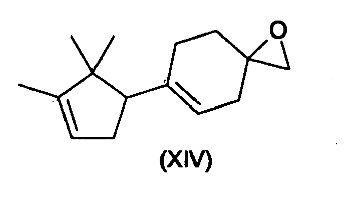
- Öffnen der Epoxidfunktion der Verbindung der Formel (XIV) und Bilden eines Aldehyds der Formel (IXa) mit R² = H 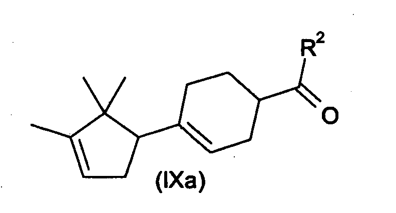
- Reduzieren des Aldehyds, so dass eine Verbindung der Formel (Ia) mit R¹, R² = H gebildet wird bzw.
nukleophile Addition einer metallorganischen Verbindung mit Methylanionencharakter an den Aldehyd, so dass eine Verbindung der Formel (Ia) mit R¹ = CH₃, R² = H gebildet wird.

14. Verbindung der Formel (XIV)

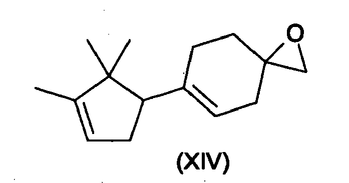

15. Verfahren zur Herstellung einer Verbindung der Formel (XIV) nach Anspruch 14 mit folgenden Schritten:
- Bereitstellen oder Herstellen einer Verbindung der Formel (XIII), 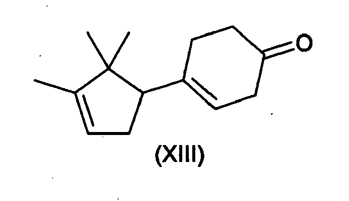
- Umwandeln der Carbonylfunktion der Verbindung der Formel (XIII) in eine Epoxidfunktion, so dass die Verbindung der Formel (XIV) resultiert.

16. Verfahren zum Erzeugen, Verstärken oder Modifizieren eines Sandelholzgeruchs in einer Mischung, mit folgenden Schritten:
- Bereitstellen einer Verbindung nach einem der Ansprüche 1 bis 3 oder einer Mischung nach einem der Ansprüche 4 bis 6,
- Bereitstellen einer Komposition sonstiger Bestandteile und
- Vermischen der Komposition sonstiger Bestandteile mit einer Menge der Verbindung oder der Mischung, die ausreicht, (a) in der resultierenden Gesamtmischung einen Sandelholzgeruch zu erzeugen, (b) einen vorhandenen Sandelholzgeruch in der Komposition sonstiger Bestandteile zu verstärken oder (c) einen vorhandenen Sandelholzgeruch in der Komposition sonstiger Bestandteile zu modifizieren.

## Claims

1. Compound of the formula (Ia) or (Ib) 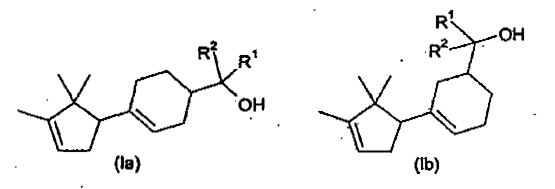 wherein in each of the formulae (Ia) and (Ib) R¹ and R² independently of each other are H or CH₃.

2. Compound according to claim 1, wherein the compound is in the form of
(a) a pure optically active enantiomer,
(b) a racemic mixture of various enantiomers of diastereomeric enantiomer pairs, or
(c) an optically active mixture of various enantiomers.

3. Compound according to claim 1 or 2, wherein:
R¹ is CH₃
and
R² is H.

4. Mixture comprising or consisting of at least one compound each of the formula (Ia) according to one of the preceding claims and of the formula (Ib) according to one of the preceding claims.

5. Mixture according to claim 4, comprising or consisting of one or more pairs of compounds of the formulae (Ia) and (Ib), wherein in the pair or the pairs the substituents R¹ and R² in the compound of the formula (Ia) have the same meaning as in the compound of the formula (Ib).

6. Mixture according to claim 5, comprising or consisting of a pair of compounds of the formulae (Ia) and (Ib),
wherein both in the compound of the formula (Ia) and in the compound of the formula (Ib)
R¹ is CH₃
and
R**²** is H.

7. Use of a compound according to one of claims 1 to 3 or of a mixture according to one of claims 4 to 6 as an odoriferous substance.

8. Odoriferous substance mixture with a sandalwood smell, comprising a compound according to one of claims 1 to 3 or a mixture according to one of claims 4 to 6 and preferably one or further conventional constituents.

9. Perfumed product comprising
- an odoriferous substance mixture according to claim 8 and
- a carrier or a substrate which is in direct contact with the odoriferous substance mixture.

10. Process for the preparation of a compound according to one of claims 1 to 3 or of a mixture according to one of claims 4 to 6 with the following steps:
- provision or preparation of a compound of the formulae (IXa) or (IXb) 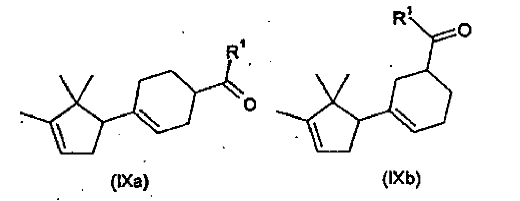 or of a mixture of a compound of the formula (IXa) with a compound of the formula (IXb),
wherein R¹ denotes H or CH₃,
- reduction of the compound or of the mixture, so that a compound according to one of claims 1 to 3 or a mixture according to one of claims 4 to 6 where R² is H is formed,
wherein R¹ has the same meaning as given for the compounds of the formulae (IXa) and (IXb), or
nucleophilic addition of an organometallic compound with a methyl anion character on to the compound or the compounds contained in the mixture, so that a compound according to one of claims 1 to 3 or a mixture according to one of claims 4 to 6 where R² is CH₃ is formed, wherein R¹ has the same meaning as given for the compounds of the formulae (IXa) and (IXb).

11. Compound of the formula (IXa) or (IXb) 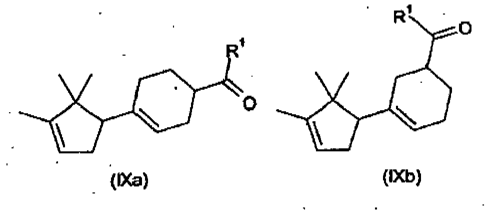 or a mixture of a compound of the formula (IXa) with a compound of the formula (IXb)
wherein R¹ denotes H or CH₃.

12. Process for the preparation of a compound of the formula (IXa) or (IXb) according to claim 11 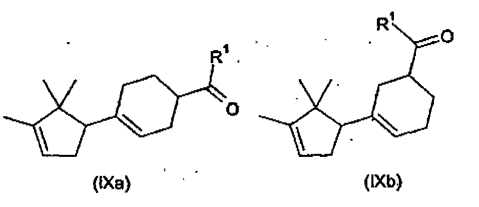 or of a mixture of a compound of the formula (IXa) with a compound of the formula (IXb),
wherein R¹ denotes H or CH₃,
with the following steps:
- conversion of a compound of the formula (X) 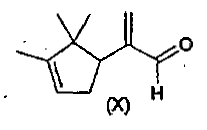 by means of an olefination reaction into a compound of the formula (XI) 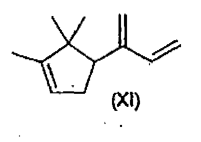
- reaction of the compound of the formula (XI) with a compound of the formula (XII) 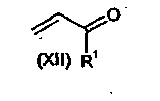 by means of a Diels-Alder reaction to give a mixture of compounds of the formulae (IXa) and (IXb), wherein in formula (XII) R¹ has the meaning given above, and optionally
- separation of the compounds of the formulae (IXa) and (IXb) from one another.

13. Process for the preparation of a compound of the formula (Ia) according to claim 1 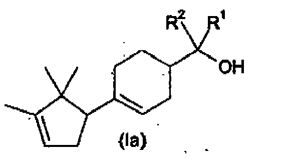 wherein
R¹ denotes H or CH₃
and
R² denotes H,
with the following steps:
- provision or preparation of a compound of the formula (XIV) 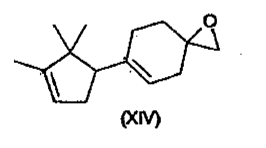
- opening of the epoxide function of the compound of the formula (XIV) and formation of an aldehyde of the formula (IXa) where R² = H 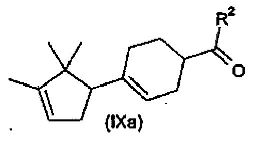
- reduction of the aldehyde, so that a compound of the formula (Ia) where R¹, R² = H is formed or
nucleophilic addition of an organometallic compound with a methyl anion character on to the aldehyde, so that a compound of the formula (Ia) where R¹ = CH₃, R² = H is formed.

14. Compound of the formula (XIV)

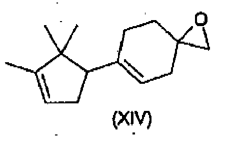

15. Process for the preparation of a compound of the formula (XIV) according to claim 14 with the following steps:
- provision or preparation of a compound of the formula (XIII) 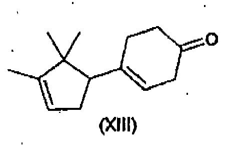
- conversion of the carbonyl function of the compound of the formula (XIII) into an epoxide function, so that the compound of the formula (XIV) results.

16. Method of generating, intensifying or modifying a sandalwood smell in a mixture with the following steps:
- provision of a compound according to one of claims 1 to 3 or of a mixture according to one of claims 4 to 6,
- provision of a composition of other constituents and
- mixing of the composition of other constituents with an amount of the compound or of the mixture which is sufficient (a) to generate a sandalwood smell in the resulting total mixture, (b) to intensify an existing sandalwood smell in the composition of other constituents or (c) to modify an existing sandalwood smell in the composition of other constituents.

## Revendications

1. Composé de la formule (Ia) ou (Ib) 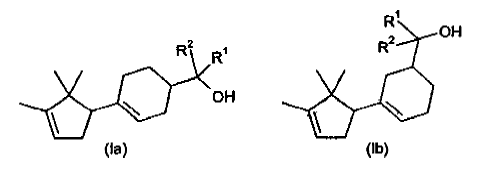 pour lequel, dans chacune des formules (Ia) et (Ib), R¹ et R² sont indépendamment l'un de l'autre H ou CH₃.

2. Composé selon la revendication 1, le composé se présentant sous la forme
(a) d'un énantiomère pur optiquement actif.
(b) d'un mélange racémique de différents énantiomères des paires d'énantiomères diastéréomères, ou
(c) d'un mélange optiquement actif de différents énantiomères.

3. Composé selon la revendication 1 ou 2, dans lequel
R¹ est CH₃
et
R² est H

4. Mélange comprenant ou consistant en au moins un composé respectivement de la formule (Ia) selon l'une quelconque des revendications précédentes et de la formule (Ib) selon l'une quelconque des revendications précédentes.

5. Mélange selon la revendication 4, comprenant ou consistant en une ou plusieurs paires de composés des formules (Ia) et (Ib), les substituants R¹ et R² dans la ou lesdites paires ayant la même signification dans le composé de la formule (Ia) que dans le composé de la formule (Ib).

6. Mélange selon la revendication 5, comprenant ou consistant en une paire de composés des formules (Ia) et (Ib), dans lequel, aussi bien dans la formule (Ia) que dans la formule (Ib),
R¹ est CH₃
et
R¹ est H.

7. Utilisation d'un composé selon l'une quelconque des revendications 1 à 3 ou d'un mélange selon l'une quelconque des revendications 4 à 6 comme parfum.

8. Mélange de parfums à odeur de bois de santal, comprenant un composé selon l'une quelconque des revendications 1 à 3 ou un mélange selon l'une quelconque des revendications 4 à 6, ainsi que, de préférence, un ou plusieurs autres composants habituels.

9. Produit parfumé, comprenant
- un mélange de parfums selon la revendication 8, ainsi que
- un support ou un substrat qui est en contact direct avec le mélange de parfums.

10. Procédé de préparation d'un composé selon l'une quelconque des revendications 1 à 3 ou d'un mélange selon l'une quelconque des revendications 4 à 6, avec les étapes suivantes :
- mise à disposition ou préparation d'un composé des formules (IXa) ou (IXb) 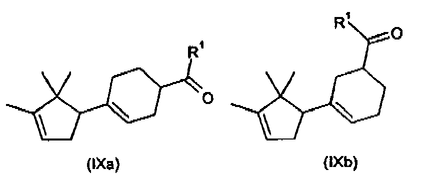 ou d'un mélange d'un composé de la formule (IXa) avec un composé de la formule (IXb),
dans lesquelles R¹ signifie H ou CH₃,
- réduction du composé ou du mélange, de telle sorte que soit formé un composé selon l'une quelconque des revendications 1 à 3, respectivement un mélange selon l'une quelconque des revendications 4 à 6, avec R² égal à H, R¹ possédant la même signification que celle spécifiée pour les composés des formules (IXa) et (IXb), respectivement addition nucléophile d'un composé organométallique à caractère méthyle-anionique au composé, ou aux composés contenus dans le mélange, de telle sorte que soit formé un composé selon l'une quelconque des revendications 1 à 3, respectivement un mélange selon l'une quelconque des revendications 4 à 6, avec R² égal à CH₃, R¹ possédant la même signification que celle spécifiée pour les composés des formules (IXa) et (IXb).

11. Composé de la formule (IXa) ou (IXb) 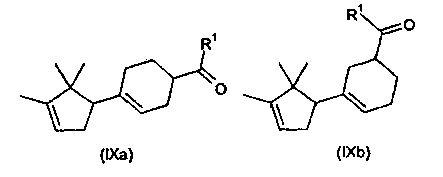 ou mélange d'un composé de la formule (IXa) avec un composé de la formule (IXb),
dans lesquelles R¹ signifie H ou CH₃.

12. Procédé de préparation d'un composé de la formule (IXa) ou (IXb) selon la revendication 11 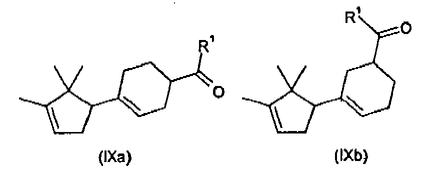 ou d'un mélange d'un composé de la formule (IXa) avec un composé de la formule (IXb),
dans lesquelles R¹ signifie H ou CH₃,
avec les étapes suivantes :
- transformation, par une réaction d'oléfination, d'un composé de la formule (X) 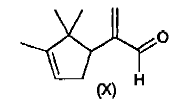 en un composé de la formule (XI) 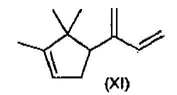
- conversion du composé de la formule (XI) avec un composé de la formule (XII) 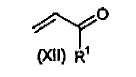 au moyen d'une réaction de Diels-Alder en un mélange de composés des formules (IXa) et (IXb), R¹ ayant dans la formule (XII) la signification spécifiée ci-dessus,
ainsi que, le cas échéant
- séparation des composés des formules (IXa) et (IXb) l'un de l'autre.

13. Procédé de préparation d'un composé de la formule (Ia) selon la revendication 1, 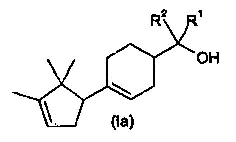 dans laquelle
R¹ signifie H ou CH₃
et
R² signifie H,
avec les étapes suivantes :
- mise à disposition ou préparation d'un composé de la formule (XIV) 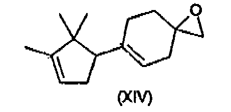
- ouverture de la fonction époxyde du composé de la formule (XIV) et formation d'un aldéhyde de la formule (IXa) avec R² = H 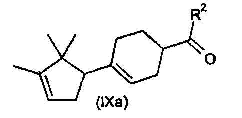
- réduction de l'aldéhyde, de telle sorte que soit formé un composé de la formule (Ia) avec R¹, R² = H, respectivement
- addition nucléophile à l'aldéhyde d'un composé organométallique à caractère méthyle-anionique, de sorte que soit formé un composé de la formule (Ia) avec R¹ = CH₃, R² = H.

14. Composé de la formule (XIV)

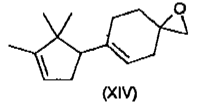

15. Procédé de préparation d'un composé de la formule (XIV) selon la revendication 14, avec les étapes suivantes:
- mise à disposition ou préparation d'un composé de la formule (XIII) 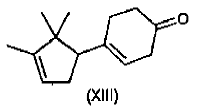
- conversion de la fonction carbonyle du composé de la formule (XIII) en une fonction époxyde, de sorte qu'il en résulte le composé de la formule (XIV).

16. Procédé de production, de renforcement ou de modification d'un parfum de bois de santal dans un mélange, avec les étapes suivantes :
- mise à disposition d'un composé selon l'une quelconque des revendications 1 à 3 ou d'un mélange selon l'une quelconque des revendications 4 à 6,
- mise à disposition d'une composition d'autres composants et
- mélangeage de la composition d'autres composants avec une quantité du composé ou du mélange qui suffit (a) à produire dans le mélange global résultant une odeur de bois de santal, (b) à renforcer une odeur de bois de santal existante dans la composition d'autres composants ou (c) à modifier une odeur de bois de santal existante dans la composition d'autres composants.
